# EUROPEAN PATENT SPECIFICATION

(11) **EP 1 320 327 B1**
(45) Date of publication and mention of the grant of the patent: **26.05.2004**
(21) Application number: 01969927.1
(22) Date of filing: 13.09.2001
(51) Int. Cl.: A61B 7/04

(54) **BIOACOUSTIC DEVICE**
BIOAKUSTISCHES GERÄT
DISPOSITIF BIOACOUSTIQUE

(30) Priority: 14.09.2000 GB 0022514
(43) Date of publication of application: 25.06.2003
(73) Proprietor: Highland Innovation Centre Limited, Inverness, IV3 8NE (GB)
(72) Inventor: MOFFOT, Ronald, James, Inverness-shire (GB)
(74) Representative: Jenkins, Peter David
(86) International application number: PCT/GB2001/004105
(87) International publication number: WO 2002/022020

(56) References cited:
- WO-A-96/13212
- DE-B- 2 836 188
- US-A- 4 071 694
- US-A- 4 438 772
- US-A- 4 440 258
- US-A- 5 638 453
- HÖK ET AL.: "Development of a wireless stethoscope for auscultatory monitoring during anaesthesia" MEDICAL & BIOLOGICAL ENGINEERING & COMPUTING, vol. 26, no. 3, - 1988 pages 317-320, XP002186649

## Description

The present invention relates to a bioacoustic device, in particular an electronic stethoscope.

It is well known that the ideal diagnostic device is non-invasive and detects the presence of diseases or conditions within the human body without the need to use, for example, X-radiation or ultrasound as these modalities may themselves lead to adverse biological changes. The standard stethoscope has such desirable properties as it detects sounds emanating from the human or animal body caused by heart sounds, respiratory sounds, and noises from bones and joints. The conventional stethoscope is limited by the fact that it is made small enough to be carried around by doctors and nurses and sometimes used as a symbol of belonging to the medical profession. From the point of view of acoustical sensitivity and thus efficiency conventional devices are not optimized. The basic instrument depends simply on an almost flat hollow horn-shaped detector, which collects sounds from the body. Connecting waveguides then carry the sounds to the ears of doctor or nurse. The surface of the detector is almost normal to the skin over a large fraction of the area and thus because of the impedance mismatch between air and metal or plastic reflects a big proportion of the incident waves. Electronic stethoscopes are an improvement on the basic acoustic version. These electronically amplify sounds so that deaf doctors are better able to hear the emanations from within the body. However current electronic devices are limited by extraneous noise from such sources as fluorescent lights, central heating pumps, surface noise caused by skin contact, and more importantly from noises arising from other sources within the body outwith with the organ or site under study.

Conventional stethoscopes have an approximately 1.5 inch (about 3.75 cm) diameter horn shaped detector, a few millimeters deep, connected to the ears of doctors by narrow air-filled pipework, which act as acoustic waveguides. The stethoscope is used primarily to listen to heart and respiratory sounds. Such a stethoscope has limited sensitivity, does not provide a record of the examination, generally requires the skin overlying the heart or lungs to be exposed and can be expensive.
Hök et al: "Development of a wireless stethoscope for ausculatory monitoring during anaesthesia" Medical & Biological Engineering & Computing, vol. 26, no. 3, - 1988 pages 317-320, XP002186649 disclose an electronic stethoscope having a conical cavity with microphone at the pointed end of the cone.

According to the invention there is provided an acoustic device comprising a cone whose diameter ranges between 5 and 10 cm at its widest, of depth between 5 and 15 mm, with a circular field effect transistor microphone of diameter between 0.5 and 2mm situated at the pointed end of the cone. Such an arrangement gives a mechanical gain of between 2500 and 10,000 in addition to any electrical gain generated by preamplifier and amplifier.

Preferably there is provided a housing machined from a solid rod of nylonite so that a cylindrical compartment lies immediately above the cone.

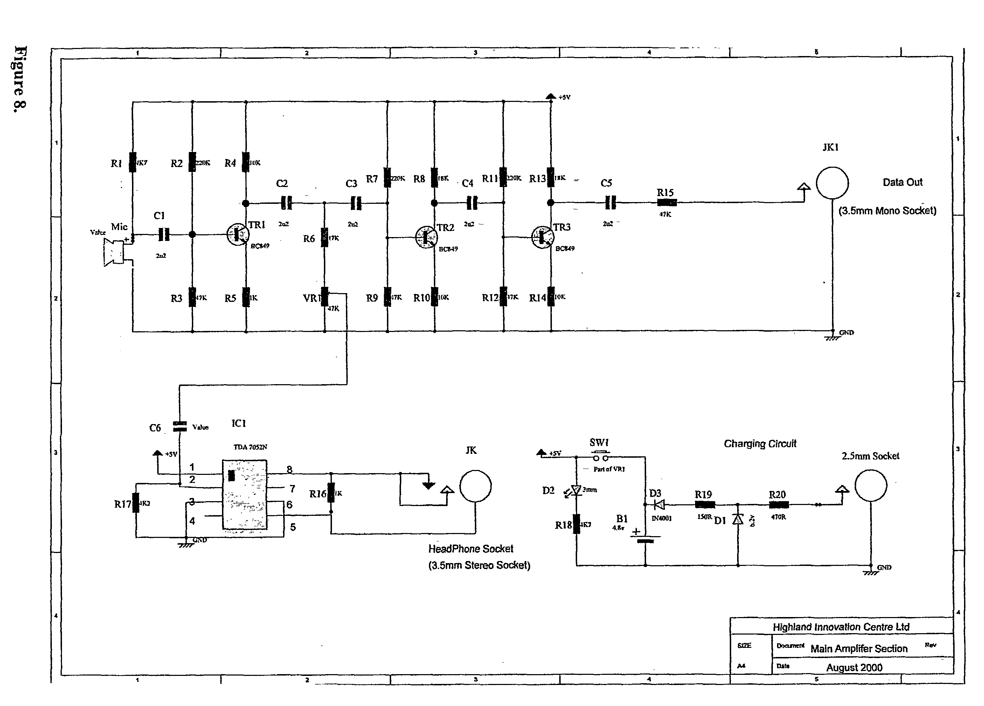

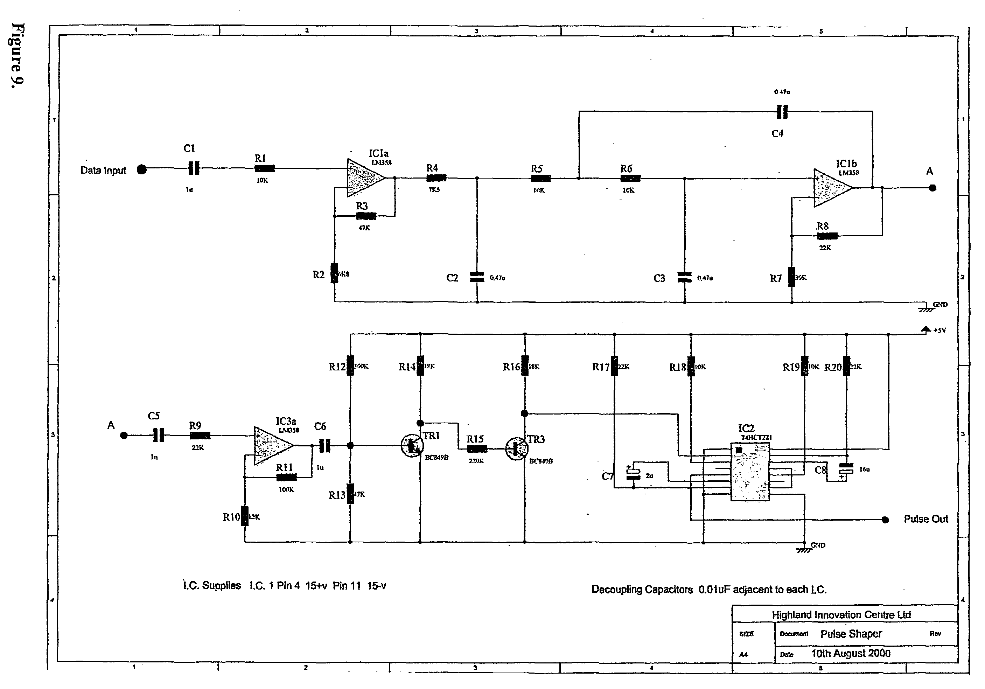

Advantageously the upper compartment also 5 to 10 cm in diameter and 3 to 7 cm deep containing a preamplifier, amplifier and circuitry as described in figures 8, 9 10 and 11.

Preferably there is provided a lid in the upper portion which contains an LCD to monitor pulse/ heart rate or other repetitive signals.

Advantageously the lid supports a data output socket for connection to a PC or other recording/analyzing device. Where there is a volume control and battery recharging socket. And where there is a control of a variable potentiometer controlling a variable frequency filter.

Optionally there is further provided a second acoustic device such that the output signal is the difference between outputs of the two devices when one is placed over a neoplastic tumour and the other on the skin but between 5 and 10 cm from it.

Preferably each device is attached to a variable arm to hold the detectors in a fixed position relative to the other while a difference signal is recorded. There is an output in the lid of the device connecting to headphones and or a loudspeaker for teaching purposes.

The present invention also provides an electronic stethoscope including an acoustic device according to the invention, the cone defining an opening in an external surface of the stethoscope for placement against the skin.

Our invention described herein overcomes these stated limitations of the prior art by greatly increasing the area of the detector of an electronic stethoscope and changing its geometry, so as to provide both substantial mechanical and electronic gain. In addition the detector and processing electronics are situated in adjacent enclosures placed on or close to the skin. An immediate advantage is that skin noise can be reduced since our device may be used through several layers of clothing. This makes it possible to avoid direct skin contact.

In a further embodiment our device utilizes two electronic stethoscopes, one overlying the source of interest and another laterally separated from it. The extent of the separation may be controlled by having the two units attached to the ends of arms which may be rotated about a common central axis. Both devices detect essentially the same background [unwanted] noise but the signal from the one closest to the sound source being investigated is detected preferentially. By subtracting the output of the secondary device from that of the first, signals from, for example, neoplastic tumours within the body caused by turbulent blood flow may be detected.

It is believed that low level sounds from cancers are associated with angiogenesis and that rapid flow in arterio-venous shunts lead to turbulence. This causes low amplitude noise.

The friable vessels composing the neovascularisation go into spasm also causing low frequency pulsations. Clearly cancer detection by non-invasive acoustic means has great potential as a diagnostic tool. In addition our invention may be applied to the study of sounds from the spine, bladder and ureters, joints, fetuses in utero and in all these instances in conditions of health and disease.

Our devices, herein described, are adapted to communicate with a personal computer so as to provide isometric displays of blood flow velocity as a function of time and amplitude.

Circuitry is included to provide an instantaneous reading of heart or pulse rate displayed on an LCD (placed on the stethoscope lid/control panel). This measurement derives from acoustic signals detected by the device.

Embodiments of the present invention are now described by way of illustration only.

Figure 1 shows an image of conical detector of stethoscope machined out of a rod of nylonite showing the FET microphone at the centre. A 10 pence coin shows scale of the device.

Figure 2 shows a diagram of a vertical section through the device showing the hollow cone 6, the microphone 5, the plastic body 2, a rubber washer 1, the electronic/ battery compartment 4, and the control panel/ lid 3. The details of the output socket and LCD are omitted for clarity.

Figure 3 shows an image of control panel of stethoscope showing phone socket, data output socket, battery recharging socket, LED warning light, and LCD panel to display heart rate.

Figure 4 shows an image demonstrating side view of stethoscope showing how battery and electronics slip into a compartment at the back of the conical detector.

Figure 5 shows a digitized signal output of stethoscope on a PC display showing amplitude as a function of time from a device placed on the clothes overlying the human heart.

Figure 6 shows the frequency spectrum of signals provided by the stethoscope from the human heart.

Figure 7 is a schematic diagram of twin detector device showing stethoscopes 14 and 17. The animal or human body is denoted by 13. The support arms are shown as 15 and 16 though the common axis and hinge is omitted for clarity. Device 14 is situated over tumour 12 which is surrounded by angiogenesis 11. Device 17 is situated away from the tumour site. Low level sounds emerge from the tumour.

Figure 8 is a circuit diagram of main amplifier section also showing battery charging circuit.

Figure 9 shows pulse shaper circuit to permit measurement of heart rate from acoustic output signals.

Figure 10 shows a circuit describing typical circuitry for display driver.

Figure 11 shows a circuit of differential amplifier section for studying low noise sources.

Since many General Practitioners now have personal computers in the examination room, we constructed a battery powered stethoscope with high sensitivity and an output to a PC so that records could be made of the examination and the frequency spectra of the sounds computed and stored for serial comparison.

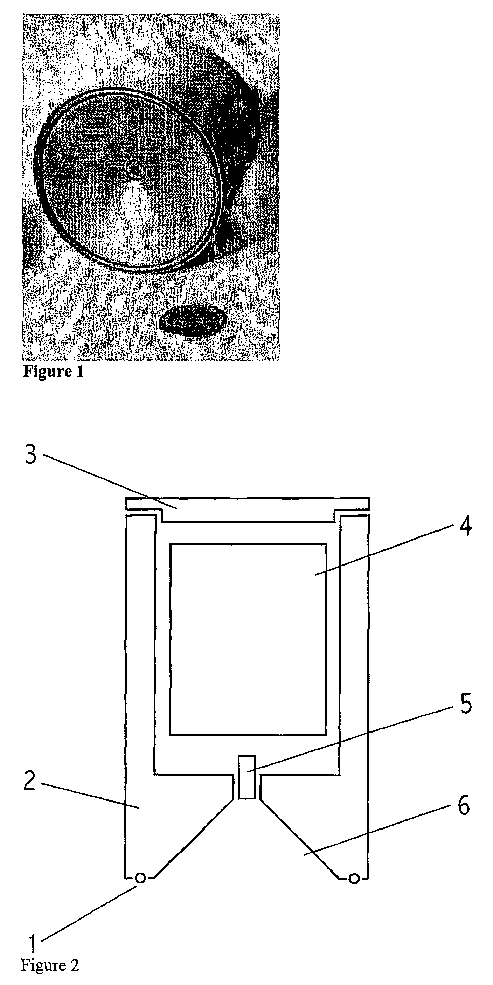

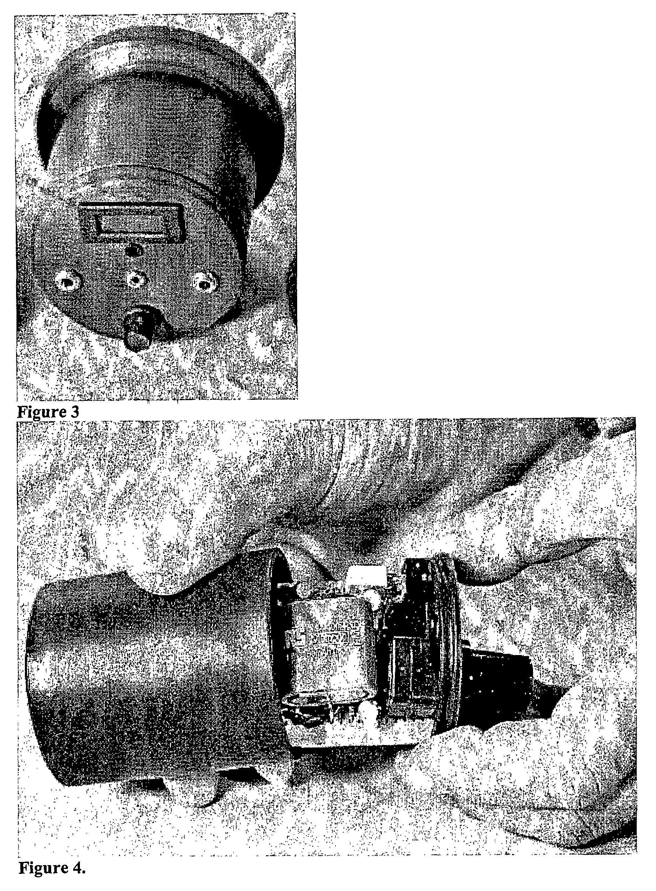

We increased the area of the detector, which is a large, thick walled, hollow circular cone, with a miniature FET [field effect] transistor microphone at its centre. The diameter of the cone, at its widest aperture, was increased to about 3.5 inches (about 8.9 cm) [Figure 1] and made deeper [about 10 mm] giving a sound collection area several times greater than that of a conventional device. The cone was machined from a solid rod of plastic so as to provide in the cylindrical space behind it a housing for the electronic amplifier, battery, filters and recharging circuitry. The full advantages of building in mechanical and electronic gain plus filtration only became apparent after construction of the first device. The wall thickness was sufficient to substantially attenuate sounds coming from external sources relative to sounds reaching the microphone directly from within the body. The large cone generates the additional mechanical gain by increasing the particle velocity amplitude of the sound wave roughly in the ratio of the catchment radius squared to that of the microphone radius squared. It obviates the need for a plastic cover placed at the skin surface as is used in some conventional stethoscopes. To keep the size of the unit to manageable physical proportions subminiature electronic components are normally utilized for the amplifier, logic and counting circuitry. A sectional diagram of the device is shown in Figure 2. An LCD display in the lid of a prototype stethoscope displays heart rate derived from the acoustic pulses and is seen in Figure 3. There are also output sockets for data transfer to a PC, for connection to headphones or loudspeaker, and a socket for recharging the internal battery. Because of the increased overall gain the new device has the ability to directly drive a loudspeaker which is advantageous because it can be used for group teaching permitting several students to listen to an examination simultaneously and to be instructed on the significance of the output sounds. The electronics and rechargeable battery fit into the space behind the conical sound detector as seen in Figure 4.

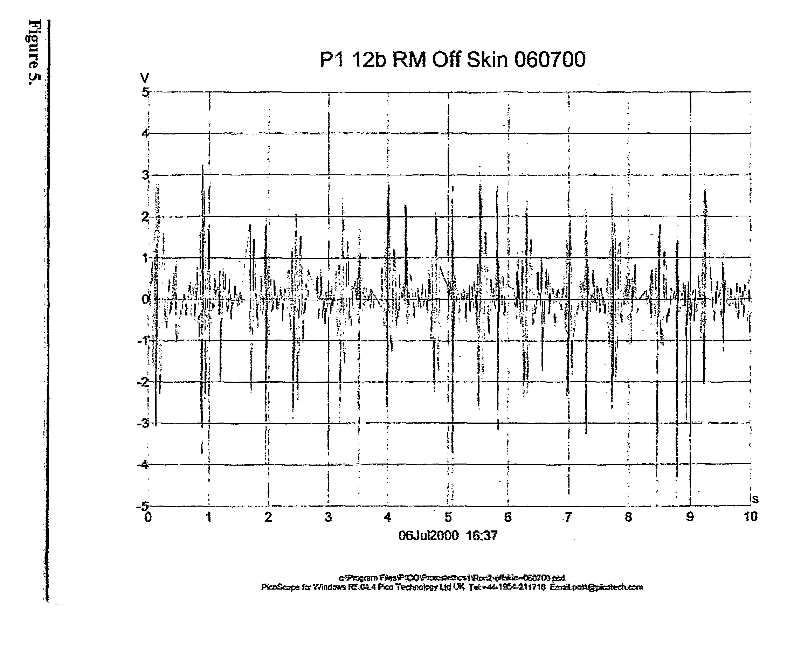

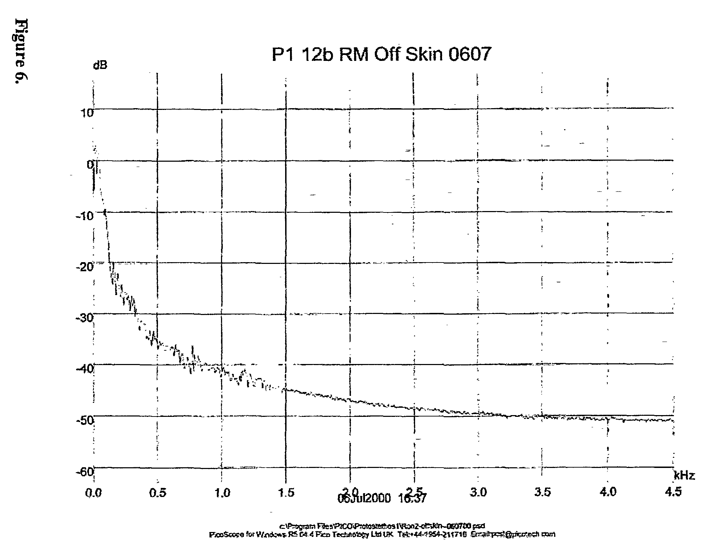

The output from the present invention is digitized and fed to a personal computer giving a signal amplitude versus time curve as seen in figure 5. When the sound source is the heart, a repeating set of signals are demonstrated at a rate of typically 1 Hz. The digitized signals may be split into frequency components by appropriate software and used to obtain a spectrum of frequencies as seen in Figure 6.

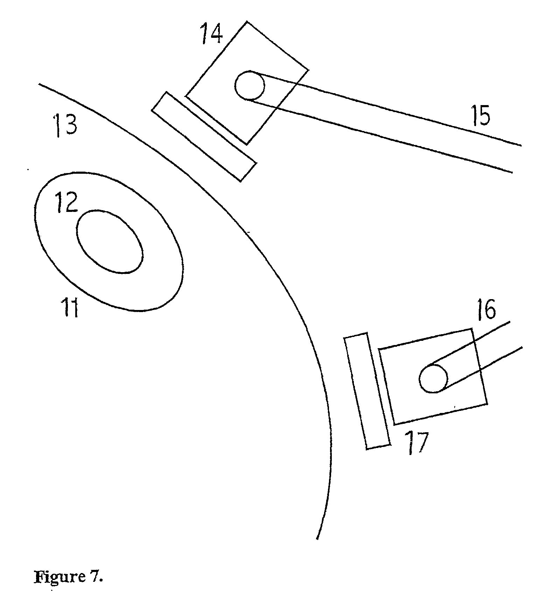

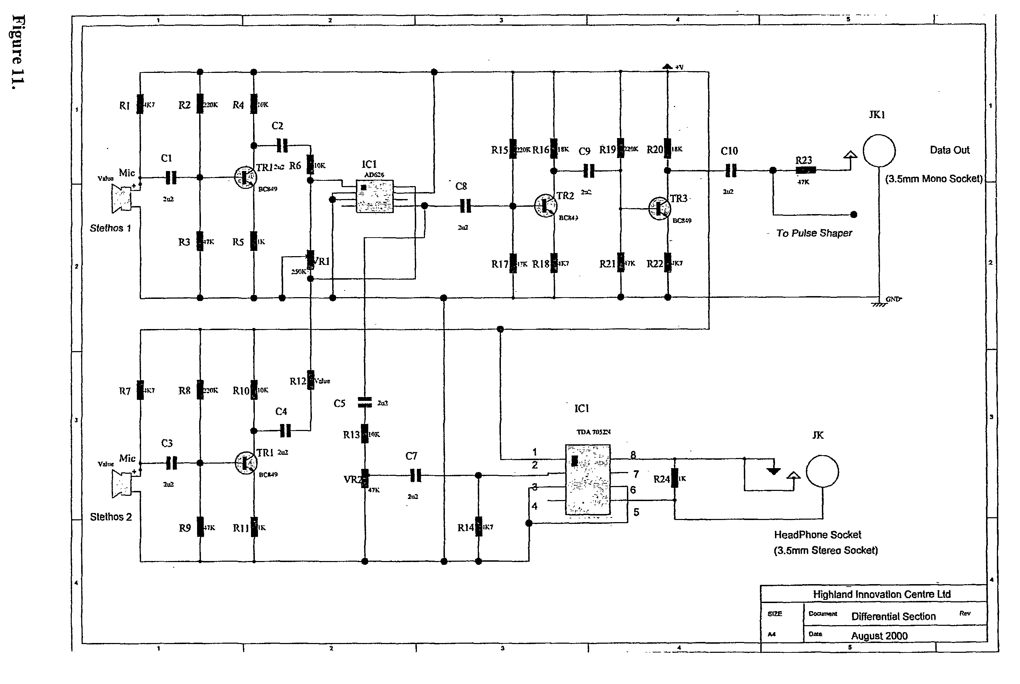

A further improvement to the detection of low level body sounds utilizes two stethoscopes each one is as described above. The output from the second device is fed to a differential amplifier situated in the first. The low level sounds emanating from a malignant tumour or other source within the body of living tissues may be demonstrated from the output signal when fed to headphones or loudspeaker. An informal drawing, Figure 7, shows the practical arrangement of twin stethoscopes for minimizing extraneous unwanted sounds. The circuitry of the differential amplifier is shown in Figure 11.

Obtaining the heart rate from acoustic signals is not straight forward because there are several pulses associated with contraction of heart muscle and from the expansion phase. After the first (major) pulse of the cardiac cycle the counting system is inhibited for a suitable period of milliseconds thus preventing false triggering of the counter circuitry. The heart rate is obtained by using either straight forward counting circuitry or indirectly using a frequency to voltage converter which was chosen to be linear down to ~0.1 Hz.

The Ultra-stethoscope unit seen in vertical section in Figure 7 is placed on the subject under investigation. The cone gathers and amplifies the sound and delivers it to the FET microphone. The signal thus obtained is electronically amplified and delivered via headphones to the operator. This signal is normally unfiltered and therefore gives a faithful reproduction of the sound source. The signal from the microphone is also amplified and routed to a data output and a heart rate counting system. An LCD provides a reading of heart rate in beats per minute. The data output signal may be fed into an analogue to digital converter which is connected to a computer which by software converts the signal to displays in either the time or frequency domains. The signals and spectra may be stored for serial comparison for example to assess the effect of drugs or other therapeutic measures on patients with heart disease.

The main amplifier circuit diagram is shown in figure 8. The circuit of the main amplifier section consists of a FET microphone complete with biasing resistor, connected to a single transistor pre-amplifier. The signal is split into two paths. The first path is routed to the main power amplifier, via a volume control on the control panel. The second path consists of a two stage amplifier which provides a raw data output. This signal is also split into two further paths one feeding the data output socket on the control panel the other providing a signal for the pulse shaper circuit. The charging circuit for the battery is also seen in Figure 8.

The circuit diagram of the pulse shaper [which is only required where a heart rate measurement is required] is seen in Figure 9 and consists of an input buffer amplifier followed by a second order low pass filter set at 30Hz. This is followed by an amplitude restoring amplifier, the output of which feeds a two transistor voltage changing section which turns the filtered signal into a logic level signal. This is fed to a one shot non-retriggerable monostable circuit, the pulse period of which is set to about 240 ms. The output is then fed to another one shot non-retriggerable monostable, the pulse period of which is set to 50 ms. This output provides the pulse for the display driver section.

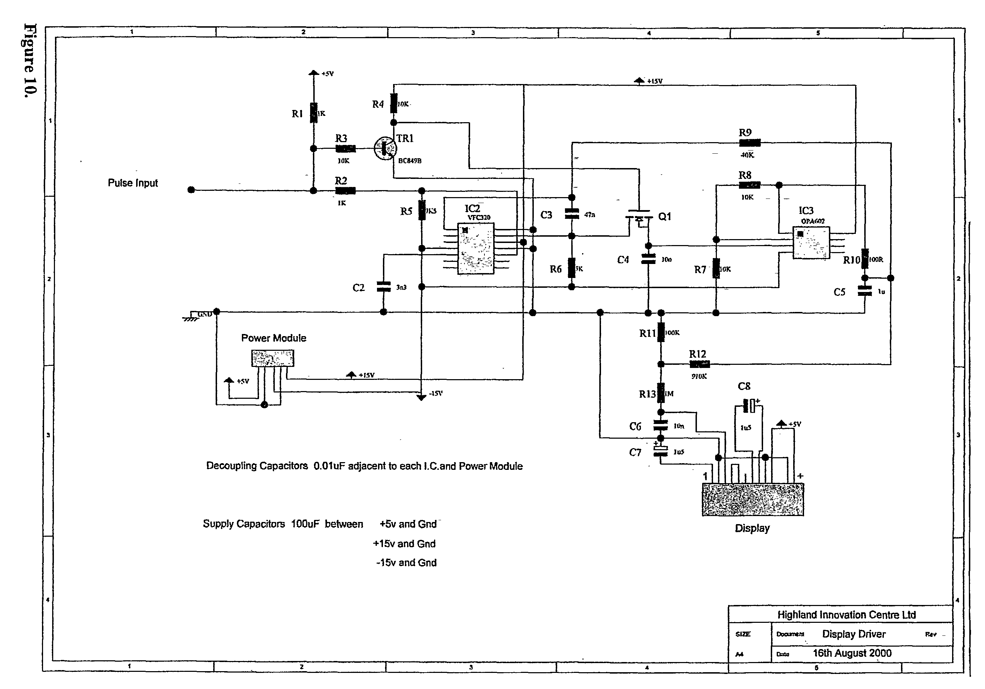

The circuit of the display driver section, seen in Figure 10, consists of a frequency to voltage converter [FVC] with a sample and hold section in the feedback loop. The pulse from the pulseshaper section triggers the FVC which provides a voltage output proportional to the input frequency. This is then scaled and fed to the panel meter on the control panel where it is displayed as the heart rate.

Surfing the internet has revealed details of conventional stethoscopes and other companies developing electronic versions. Details of their development costs and the capabilities of the versions developed by competitors are easily found. However our device is, as far as we know, the only one to make use of increased mechanical and electronic gain and to incorporate the following features: [1] can be used through 5 layers of clothing [2] electronic filtration for high and low frequency sounds [3] PC output and software for displaying data [4] frequency analysis of sound data. [5] LCD display of heart (pulse) rate. In addition our device will be developed for other uses such as sounds from arthritic joints, from fractures, fetal heart detection, tumour detection and others clinical purposes.

One problem with stethoscopes is that extraneous noises, not of specific interest to the operator, are also amplified making it difficult to identify sound from very low level sources such as tumours. To overcome this problem we have developed a dual stethoscope system. The device consists of two detector units [each one similar to that seen in Figure 2]. In our preferred embodiment the devices are attached to two arms pivoted about a central axis. The arrangement is seen diagrammatically in Figure 7. The signals from the two microphones are fed to a differential amplifier section inside one of the units, where the resultant signal is the difference between the two signals. This attenuates common extraneous signals, generated both internally and externally, thus permitting low level diagnostic sounds to be detected and analyzed. Provision is made for the operator to select either the differential signal or that from either microphone. The signal is then amplified and delivered to the operator via headphones. The unit is fitted with a variable filtering system which may be selected and manipulated by the operator to further enhance selected frequency bands of the sound under investigation. A data output is also provided and the operator may choose which signal is fed to the data output socket. Provision is also included so that the operator may select either the raw signal data or the filtered signal to be routed to the data socket. This is then fed to an analogue to digital converter which is connected to a computer which via software provides a display of signal amplitude versus time. Also a frequency spectrum of the signal may be obtained and stored for comparison. This is valuable for studying blood flow through angiogenesis around cancers during treatment with antiangiogenic drugs.

The HIC device, the Ultra-stethoscope has a number of market advantages.
[1] It is easier to use and allows the doctor or nurse to vary the sound level.
[2] It can power a loudspeaker for use in teaching permitting a group of students to listen to an examination and have the salient features explained to them.
[3] The device allows serial comparison of examinations by connecting it to a PC.
[4] Frequency analysis of sound data can be carried out.
[5] The device does not require the patient to undress as it works well through many layers of clothing. Muslim patients may be attracted by this feature.
[6] It automatically measures and displays heart rate.
[7] There are many noises generated by the bodies of both well and sick people where the significance is not understood but which might be diagnostically important.
[8] Non-invasive testing without putting radiation or ultrasound into the body may be valuable for studying the fetus in utero.
[9] Several versions of the device whose estimated selling price ranges from £40 up to around £500, mean that there can be a large potential market in both developed and developing countries. The price depends on features included and the sale of a separate software package.

This invention has potential importance of acoustic detection in medicine and other fields such as insect infestation testing.

The figures attached show the prototypes, the electronic circuitry, the output in time and frequency domains, and the move to subminiature components. In the 'twin acoustic detector' embodiment unwanted background noise is subtracted off the primary signal. If sounds consequent upon the transition from smooth to turbulent blood flow is to be detected (e.g. from arterio-venous shunts associated with cancers) then subtraction of background sounds away from the main sound source offers the best chance of success in adapting the devices as detectors of neoplastic tumours. We are convinced from our own scientific and clinical knowledge and from market research that the ultra-stethoscope offers the opportunity to build a successful business with a range of products having a good short and excellent long term future.

## Claims

1. An acoustic device comprising a cone (6) and a microphone (3) situated at the pointed end of the cone (6) **characterised in that** the diameter of the cone (6) ranges between 5 and 10 cm at its widest, of depth between 5 and 15 mm, and the microphone (5) is a circular field effect transistor microphone (5) of diameter between 0.2 and 2mm.

2. An acoustic device as claimed in claim 1, wherein there is provided a housing (2) machined from a solid rod of plastics so that a cylindrical compartment (4) lies immediately above the cone (6).

3. An acoustic device as claimed in claims 1 or 2, further comprising a compartment containing a preamplifier, amplifier and circuitry.

4. An acoustic device as claimed in claims 1, 2 or 3, wherein there is provided a lid (3) in the upper portion which contains an LCD to display pulse heart rate or other repetitive signals.

5. An acoustic device as claimed in claim 4, wherein the lid (3) supports a data output socket for connection to a PC or other recording/analysing device.

6. An acoustic device as claimed in any foregoing claim further comprising a volume control and battery recharging socket.

7. An acoustic device as claimed in any foregoing claim further comprising a control of a variable potentiometer controlling a variable frequency filter.

8. An acoustic device according to any foregoing claim wherein there is an output of the device for connecting to headphones and/or a loudspeaker.

9. The combination of two acoustic devices (14,17) as claimed in any foregoing claim electrically connected together to produce a common output signal which is the difference between the individual outputs of the two devices (14,17) when one is placed over a neoplastic tumour (12) and the other on the skin but between 15 and 10 cm from it.

10. The combination as claimed in claim 9, wherein each device is attached to a variable arm (15,16) to hold the detectors in a fixed position relative to the other while a different signal is recorded.

11. An electronic stethoscope including an acoustic device according to any one of claims 1 to 8, the cone defining an opening in an external surface of the stethoscope for placement against the skin, the stethoscope providing a mechanical gain of from 2500 to 10000.

12. An electronic stethoscope according to claim 11 wherein the cavity is conical.

13. An electronic stethoscope according to claim 11 or claim 12 wherein the opening has a width of from 5 to 10 cm, the cavity has a depth of from 5 to 15 mm and the electronic microphone has a width of from 0.5 to 2 mm.

## Patentansprüche

1. Akustische Vorrichtung umfassend einen Konus (6) und ein Mikrophon (3), welches an dem spitz zulaufenden Ende des Konus (6) angeordnet ist, **dadurch gekennzeichnet, daß** der Durchmesser des Konus (6) sich zwischen 5 und 10 cm bewegt, wo er am weitesten ist, von einer Tiefe zwischen 5 und 15 mm ist, und daß das Mikrophon (5) ein ringförmiges Feldeffekttransistormikrophon (5) mit einem Durchmesser zwischen 0,2 und 2 mm ist.

2. Akustische Vorrichtung wie in Anspruch 1 beansprucht, wobei ein Gehäuse (2) vorgesehen ist, welches maschinell aus einem massiven Kunststoffstab hergestellt ist, so daß ein zylindrisches Abteil (4) direkt oberhalb des Konus (6) liegt.

3. Akustische Vorrichtung wie in Anspruch 1 oder 2 beansprucht, ferner umfassend ein Abteil, welches einen Vorverstärker, einen Verstärker und eine Schaltungsanordnung enthält.

4. Akustische Vorrichtung wie in Anspruch 1, 2 oder 3 beansprucht, wobei ein Deckel in dem oberen Teil vorgesehen ist, welcher ein LCD enthält, um Puls/Herzfrequenz oder andere sich wiederholende Signale anzuzeigen.

5. Akustische Vorrichtung wie in Anspruch 4 beansprucht, wobei der Deckel (3) einen Datenausgabesockel zum Verbinden mit einem PC oder einer anderen Aufzeichnungs-/Analysevorrichtung trägt.

6. Akustische Vorrichtung wie in einem der vorhergehenden Ansprüche beansprucht, ferner umfassend eine Lautstärkesteuer- und/oder -regelung und einen Batterieladesockel.

7. Akustische Vorrichtung wie in einem der vorhergehenden Ansprüche beansprucht, ferner umfassend eine Steuer- und/oder Regelung eines variablen Potentiometers, welches einen variablen Frequenzfilter steuert und/oder regelt.

8. Akustische Vorrichtung nach einem der vorhergehenden Ansprüche, wobei ein Ausgang der Vorrichtung zur Verbindung mit Kopfhörern und/oder einem Lautsprecher vorgesehen ist.

9. Kombination von zwei akustischen Vorrichtungen (14, 17) wie sie in einem der vorhergehenden Ansprüche beansprucht sind, welche elektrisch miteinander verbunden sind, um ein gemeinsames Ausgangssignal zu erzeugen, welches die Differenz zwischen den individuellen Ausgängen der beiden Vorrichtungen (14, 17) ist, wenn eine über einem neoplastischen Tumor (12) und die andere auf der Haut, aber zwischen 15 und 10 cm davon angeordnet ist.

10. Kombination wie in Anspruch 9 beansprucht, wobei jede Vorrichtung an einen variablen Arm (15, 16) angegliedert ist, um die Detektoren in einer festen Stellung relativ zueinander zu halten, während ein Differenzsignal aufgezeichnet wird.

11. Elektronisches Stethoskop, umfassend eine akustische Vorrichtung nach einem der Ansprüche 1 bis 8, wobei der Konus eine Öffnung in einer äußeren Oberfläche des Stethoskops definiert zur Plazierung an die Haut, wobei das Stethoskop eine mechanische Verstärkung von 2500 bis 10.000 bereitstellt.

12. Elektronisches Stethoskop nach Anspruch 11, wobei der Hohlraum konisch ist.

13. Elektronisches Stethoskop nach Anspruch 11 oder Anspruch 12, wobei die Öffnung eine Weite von 5 bis 10 cm aufweist, der Hohlraum eine Tiefe von 5 bis 15 mm aufweist und das elektronische Mikrophon eine Weite von 0,5 bis 2 mm aufweist.

## Revendications

1. Dispositif acoustique comprenant un cône (6) et un microphone (5) situé à l'extrémité pointue du cône (6), **caractérisé en ce que** le diamètre du cône (6) est compris entre 5 et 10 cm dans sa plus grande largeur, et sa profondeur entre 5 et 15 mm, le microphone (5) étant un microphone circulaire (5) à transistor à effet de champ d'un diamètre compris entre 0,2 et 2 mm.

2. Dispositif acoustique selon la revendication 1, dans lequel il est prévu un boîtier (2) usiné à partir d'une tige pleine de matière plastique de sorte qu'un compartiment cylindrique (4) se trouve immédiatement au-dessus du cône (6).

3. Dispositif acoustique selon la revendication 1 ou 2, comprenant en outre un compartiment contenant un préamplificateur, un amplificateur et des circuits.

4. Dispositif acoustique selon la revendication 1, 2 ou 3, dans lequel il est prévu un couvercle (3) dans la partie supérieure qui contient un affichage à cristaux liquides pour afficher un rythme cardiaque en impulsion ou autre signal répétitif.

5. Dispositif acoustique selon la revendication 4, dans lequel le couvercle (3) porte une douille de sortie de données pour connexion à un ordinateur personnel ou autre dispositif d'enregistrement/analyse.

6. Dispositif acoustique selon l'une quelconque des revendications précédentes, comprenant en outre une commande d'amplitude et une douille de rechargement de batterie.

7. Dispositif acoustique selon l'une quelconque des revendications précédentes, comprenant en outre une commande d'un potentiomètre variable commandant un filtre à fréquence variable.

8. Dispositif acoustique selon l'une quelconque des revendications précédentes, dans lequel il est prévu une sortie pour connexion à des écouteurs et/ou un haut-parleur.

9. Combinaison de deux dispositifs acoustiques (14, 17) selon l'une quelconque des revendications précédentes connectés électriquement l'un à l'autre pour fournir un signal de sortie commun qui est la différence entre les sorties individuelles des deux dispositifs (14, 17) quand l'un est placé sur une tumeur néoplastique (12) et l'autre sur la peau mais à entre 15 et 10 cm du premier.

10. Combinaison selon la revendication 9, dans laquelle chaque dispositif est fixé à un bras variable (15, 16) pour tenir les détecteurs à une position fixe par rapport à l'autre tandis qu'un signal différent est enregistré.

11. Stéthoscope électronique comprenant un dispositif acoustique selon l'une quelconque des revendications 1 à 8, la surface externe du cône du stéthoscope étant destinée à être placée contre la peau, le stéthoscope fournissant un gain mécanique de 2500 à 10000.

12. Stéthoscope électronique selon la revendication 11, dans lequel la cavité est conique.

13. Stéthoscope électronique selon la revendication 11 ou 12, dans lequel l'ouverture a une largeur d'environ 5 à 10 cm, la cavité a une profondeur d'environ 5 à 15 mm et le microphone électronique a une largeur d'environ 0,5 à 2 mm.
